# EUROPEAN PATENT SPECIFICATION

(11) **EP 2 017 607 B1**
(45) Date of publication and mention of the grant of the patent: **03.10.2012**
(21) Application number: 07741962.0
(22) Date of filing: 19.04.2007
(51) Int. Cl.: G01N 27/327, G01N 27/416, G01N 33/543, C12Q 1/00

(54) **BIOSENSOR**
BIOSENSOR
BIOCAPTEUR

(30) Priority: 19.04.2006 JP 2006116175
(43) Date of publication of application: 21.01.2009
(60) Divisional application: 11158309.2
(73) Proprietor: Panasonic Corporation, Kadoma-shi Osaka 571-8501 (JP)
(72) Inventor: NAKAYAMA, Junko, Shiromi, Chuo-ku, Osaka-shi, Osaka 540-6207 (JP); TAKAHARA, Yoshifumi, Shiromi, Chuo-ku, Osaka-shi, Osaka 540-6207 (JP); YAMANISHI, Eriko, Shiromi, Chuo-ku, Osaka-shi, Osaka 540-6207 (JP); ITOH, Yoshihiro, Shiromi, Chuo-ku, Osaka-shi, Osaka 540-6207 (JP)
(74) Representative: Pautex Schneider, Nicole Véronique
(86) International application number: PCT/JP2007/058526
(87) International publication number: WO 2007/123178

(56) References cited:
- EP-A1- 1 785 483
- WO-A2-2004/038401
- JP-A- 2003 279 525
- JP-A- 2006 017 720
- LAURINAVICIUS V ET AL: "OXYGEN INSENSITIVE GLUCOSE BIODENSOR BASED ON PQQ-DEPENDENT GLUCOSE DEHYDROGENASE" ANALYTICAL LETTERS, NEW YORK, NY, US, vol. 32, no. 2, 1 January 1999 (1999-01-01), pages 299-316, XP009070271 ISSN: 0003-2719
- TSUJIMURA S ET AL: "Novel FAD-Dependent Glucose Dehydrogenase for a Dioxygen-Insensitive Glucose Biosensor", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 70, no. 3, 1 March 2006 (2006-03-01), pages 654-659, XP003004577, ISSN: 0916-8451, DOI: DOI:10.1271/BBB.70.654

## Description

### TECHNICAL FIELD

The present invention relates to a biosensor for measuring the concentration of a specific component in a sample solution.

### BACKGROUND ART

A biosensor is a sensor which utilizes the molecule identifying abilities of biological materials such as microorganisms, enzymes, and antibodies to apply the biological materials as molecule recognition elements. To be specific, the biosensor utilizes a reaction which occurs when an immobilized biological material recognizes a target specific component, such as oxygen consumption by respiration of a micro-organism, an enzyme reaction, or luminescence.

Among biosensors, enzyme sensors have been advanced in practical applications, and for example, enzyme sensors for glucose, lactic acid, cholesterol, lactose, uric acid, urea, and amino acid are utilized in medical measurement and food industry. An enzyme sensor reduces an electron acceptor by an electron generated by a reaction between an enzyme and a substrate included in a sample solution as a specimen, and a measurement device electrochemically measures the oxidation-reduction quantity of the electron acceptor, thereby to perform quantitative analysis of the specimen.

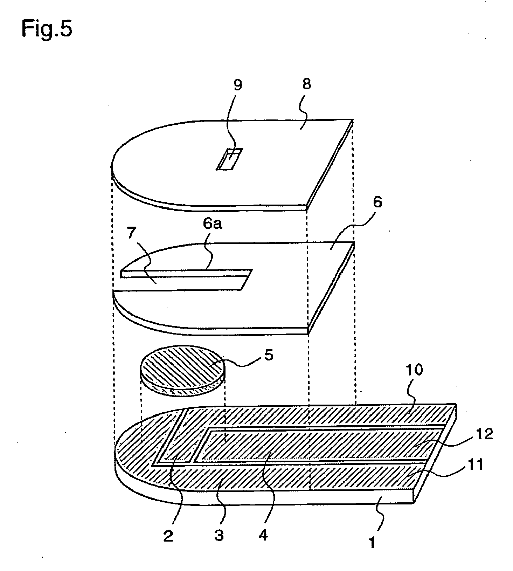

Figure 5 shows an exploded perspective view of a three-electrode-system biosensor as an example of such biosensor.

The biosensor shown in figure 5 is fabricated as follows. After an electric conductive layer is formed on an insulating substrate 1 by a sputtering deposition method or a screen printing method, slits are formed using laser or the like to produce a working electrode 2, a counter electrode 3, and a detection electrode 4, and then a reagent layer 5 including an enzyme which reacts with a specific component in a sample solution, and an electron carrier is formed on these electrodes. Further, a spacer 6 having a notch 6a and a cover 8 are bonded together onto the reagent layer 5 and the electrodes 2, 3, and 4, thereby forming a cavity 7 into which the sample solution is supplied. While supply of the sample solution from the cavity 7 into the biosensor is realized by a capillary phenomenon, smooth supply of the sample solution is realized by providing the cover 8 with an air hole 9 for letting the air in the cavity 7 out of the biosensor.

When the sample solution is applied to an inlet of the cavity 7 of thus configured biosensor, the sample solution is supplied from the inlet of the cavity 7 into the cavity 7 by the capillary phenomenon, and when it reaches the position of the reagent layer 5, the specific component in the sample solution reacts with the reagent included in the reagent layer 5. The amount of change in current which occurs due to this reaction is read with an external measurement device which is connected through the leads 10, 11, and 12 of the working electrode 2, the counter electrode 3, and the detection electrode 4, respectively. The read current value is converted into the concentration of the specific component to determine the quantity of the specific component in the sample solution.
Document EP 1 785 483 describes a glucose biosensor comprising a PQQ-dependent glucose dehydrogenase as an enzyme and an electrochemical cell.
Document WO 2004/038401 describes a conventional glucose biosensor.
Non patent literature, Laurinavicius V et al: "Oxygen insensitive glucose biosensor based on PQQ dependent dehydrogenase" Analytical letters, vol. 32, No. 2, 1999, pages 299-316 describes a glucose biosensor comprising a PQQ-dependent glucose dehydrogenase as an enzyme
Patent Document 1: Japanese Published Patent Application No. 2000-171428
Patent Document 2: Japanese Published Patent Application No. 2001-343350
Patent Document 3: Japanese Published Patent Application No. 2002-207022

### DISCLOSURE OF THE INVENTION

### PROBLEMS TO BE SOLVED BY THE INVENTION

However, the conventional biosensor has a problem that a correct inspection result cannot be obtained due to an influence of hematocrit if the sample solution is blood. Especially an enzyme sensor for glucose is often used for measurement at the time of insulin injection before meal or evaluation for low blood sugar, and there is a possibility of inviting excessive administration of insulin or missing of blood sugar level if the glucose concentration is displayed higher than the actual value due to the influence by hematocrit. Therefore, a highly precise biosensor which is not affected by the influence by hematocrit when the sample solution is blood is desired.

Further, the conventional biosensor has a problem that a correct inspection result cannot be obtained due to an influence by ambient temperature. Although the inspection result is corrected using a temperature control device or the like to resolve this problem, wrong correction might be performed if the temperature control device cannot respond to a rapid temperature change and falsely recognizes the temperature, and a correct inspection result cannot be obtained. Accordingly, a biosensor which is hardly affected by the influence by ambient temperature is demanded.

The present invention is made to solve the above-described problems and has for its object to provide a highly precise biosensor which can avoid the influence by hematocrit and the influence by ambient temperature.

### MEASURES TO SOLVE THE PROBLEMS

In order to solve the above-described problems, the present invention provides a biosensor as defined in claim 1.

### BRIEF DESCRIPTION OF THE DRAWINGS

Figure 1 is a diagram illustrating the influence by hematocrit to sensor response characteristics in the case where BSA is added to a reagent layer in a biosensor according to a first example of the present invention.
Figure 2 is a diagram illustrating the influence by temperature to sensor response characteristics in the case where BSA is added to the reagent layer in the biosensor according to the first example
Figure 3 is a diagram illustrating the influence by hematocrit to sensor response characteristics in the case where BSA is added to a reagent layer in a biosensor according to a reference example of the present invention,
Figure 4 is a diagram illustrating the influence by temperature to sensor response characteristics in the case where BSA is added to the reagent layer in the biosensor according to the reference example.
Figure 5 is an exploded perspective view of a conventional three-electrode-system biosensor.

### DESCRIPTION OF REFERENCE NUMERALS

- 1 ...: substrate
- 2 ...: working electrode
- 3 ...: counter electrode
- 4 ...: detection electrode
- 5 ...: reagent layer
- 6 ...: spacer
- 6a ...: notch
- 7 ...: cavity
- 8 ...: cover
- 9 ...: air hole
- 10,11,12...: leads

### BEST MODE TO PERFORM THE INVENTION

Hereinafter, preferred example of the present invention will be described with reference to the drawings. In the example of the present invention described hereinafter, an enzyme sensor which adopts an enzyme as a molecular identification element that reacts specifically with a specific component in a sample solution will be exemplified.

### (First example)

A biosensor according to a first example of the present invention will be described.

The biosensor of this first example is characterized by that a solubilized protein is added to the reagent layer 5 of the biosensor shown in figure 5. In this first example glucose dehydrogenase having flavin adenine dinucleotide as a coenzyme (hereinafter referred to as FAD-GDH) is used as an enzyme. Further, bovine serum albumin (hereinafter referred to as BSA) is used as a solubilized protein.

Hereinafter, the function and effect of this first example will be described.

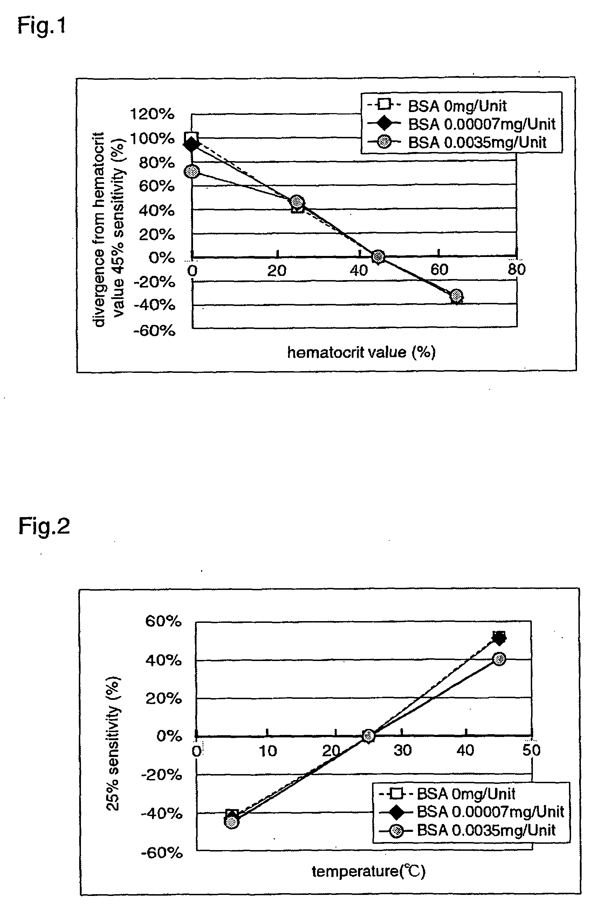

Figure 1 is a diagram illustrating the influence by hematocrit to sensor response characteristics when BSA is added to the reagent layer 5 in the biosensor of the first example. In figure 1, the ordinate shows the divergence from the sensor sensitivity when the hematocrit value is 45%, and the abscissa shows the hematocrit value.

Whole blood having a glucose concentration adjusted to 350mg/dL is used as a sample solution. Further, the enzyme amount of FAD-GDH is 2U (unit) per one sensor.

The sensor response value is measured with the concentration of BSA added to the reagent layer 5 which constitutes the biosensor of the first example being varied from 0 to 0.0035mg per enzyme 1U, in other words, from 0 to 0.007mg per one sensor in the reagent solution.

As the result of measurement, in the concentration range where the hematocrit value is 25 to 65%, no change is found in the sensor response value even when BSA is added. On the other hand, in the low concentration range where the hematocrit value is 25% and below, the influence by hematocrit to the sensor response characteristics is reduced according to the additive amount of BSA.

In this way, when FAD-GDH and BSA are contained in the reagent layer 5, BSA addition effect is recognized in the low concentration range where the hematocrit value is 0 to 25%, while no BSA addition effect is recognized in the concentration range where the hematocrit value is 25% and above.

Figure 2 is a diagram illustrating the influence by temperature to the sensor response characteristics when BSA is added to the reagent layer 5 in the biosensor of the first example. In figure 2, the ordinate shows the divergence from the sensitivity at temperature 25°C, and the abscissa shows the temperature.

Whole blood having a glucose concentration adjusted to 350mg/dL is used as a sample solution. Further, the enzyme amount of FAD-GDH is 2U per one sensor.

The sensor response characteristics are measured with the concentration of BSA added to the reagent layer 5 which constitutes the biosensor of the first example being varied from 0 to 0.0035mg per enzyme 1U, in other words, from 0 to 0.007mg per one sensor in the reagent solution.

As the result of measurement, in the temperature range of 5 to 25°C, no change is found in the sensor response value even when BSA is added. On the other hand, in the high temperature range of 25°C and above, the influence by temperature to the sensor response characteristics is reduced according to the additive amount of BSA.

In this way, when FAD-GDH and BSA are contained in the reagent layer 5, BSA addition effect is recognized in the temperature range higher than 25°C, while no BSA addition effect is recognized in the temperature range of 25°C and below.

Consequently, it can be determined that the characteristics of both the hematocrit and the temperature can be enhanced when BSA is included in the reagent layer or 5 by 0.0035mg per enzyme 1U or by 0.007mg per one sensor.

According to the biosensor of this first example, since a large amount of BSA is contained in the reagent layer including the enzyme FAD-GDH, the influences by hematocrit and temperature to the sensor response characteristics can be improved. Reference example

A biosensor according to a reference example of the present invention will be described.

The biosensor of this reference example is characterized by that a solubilized protein is added to the reagent layer 5 of the biosensor shown in figure 5. In this reference example glucose dehydrogenase having pyrrolo-quinoline quinone as a coenzyme (hereinafter referred to as PQQ-GDH) is used as an enzyme. Further, bovine serum albumin (hereinafter referred to as BSA) is used as a solubilized protein.

Hereinafter, the function and effect of this reference example will be described.

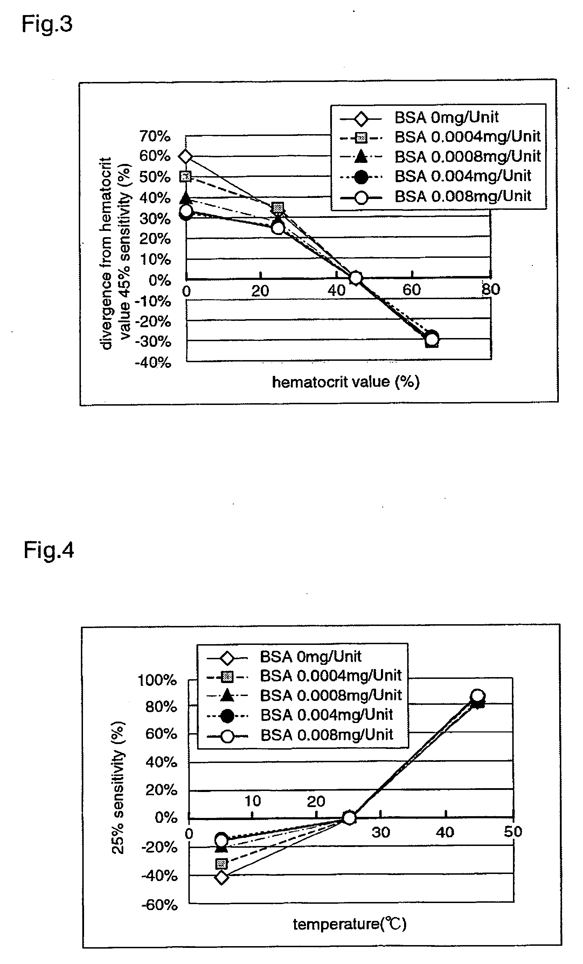

Figure 3 is a diagram illustrating the influence by hematocrit to sensor response characteristics when BSA is added to the reagent layer 5 in the biosensor of the reference example. In figure 3, the ordinate shows the divergence from the sensor sensitivity when the hematocrit value is 45%, and the abscissa shows the hematocrit value.

Whole blood having a glucose concentration adjusted to 350mg/dL is used as a sample solution. Further, the enzyme amount of PQQ-GDH is 1.7U per one sensor.

The sensor response value is measured with the concentration of BSA added into the reagent layer 5 which constitutes the biosensor of the reference example being varied from 0 to 0.008mg per enzyme 1U, in other words, from 0 to 0.014mg per one sensor in the reagent solution.

As the result of the measurement, no BSA addition effect to the sensor response value is recognized in the region where the hematocrit value is 25 to 65%. On the other hand, in the low concentration range where the hematocrit value is 25% and below, the influence by hematocrit to the sensor response characteristics is reduced according to the additive amount of BSA.

In this way, when PQQ-GDH and BSA are contained in the reagent layer 5, BSA addition effect is recognized in the low concentration range where the hematocrit value is 0 to 25%, while no BSA addition effect is recognized in the concentration range where the hematocrit value is 25% and above.

Figure 4 is a diagram illustrating the influence by temperature to the sensor response characteristics when BSA is added to the reagent layer 5 in the biosensor of the reference example. In figure 2, the ordinate shows the divergence from the sensitivity at temperature 25°C, and the abscissa shows the temperature.

Blood plasma having a glucose concentration adjusted to 350mg/dL is used as a sample solution. The enzyme amount of PQQ-GDH is 1.7U per one sensor.

The sensor response characteristics are measured with the concentration of BSA added to the reagent layer 5 which constitutes the biosensor of the reference example being varied from 0 to 0.008mg per enzyme 1U, in other words, from 0 to 0.014mg per one sensor in the reagent solution.

As the result of measurement, in the temperature range of 25 to 45°C, no change is found in the sensor response value even when BSA is added. On the other hand, in the low temperature range of 250°C and below, the influence by temperature to the sensor response characteristics is reduced according to the additive amount of BSA.

In this way, when PQQ-GDH and BSA are contained in the reagent layer 5, BSA addition effect is recognized when the temperature is lower than 25°C, while no BSA addition effect is recognized when the temperature is 25°C or above.

Consequently, it can be determined that the characteristics of both the hematocrit and the temperature can be enhanced when BSA is included in the reagent layer 5 by 0.0004 to 0.008mg per enzyme 1U or by 0.0007 to 0.014mg per one sensor. Further, since the effect is not changed when the BSA is added by more than 0.004mg per enzyme 1U or by more than 0.007mg per one sensor, it can be determined that the optimum value of BSA is 0.004mg per enzyme 1U or 0.007mg per one sensor.

According to the biosensor of this reference example, since a large amount of BSA is contained in the reagent layer including the enzyme PQQ-GDH, the influences by hematocrit and temperature to the sensor response characteristics can be improved.

While in the first example FAD-GDH is adopted as the enzymes in the reagent layer 5, there may be adopted other enzymes used for clinical inspection, such as cholesterol oxidase, cholesterol esterase, cholesterol dehydrogenase, lipoprotein lipase, catalase, peroxidase, lactate oxidase, lactate dehydrogenase, urease, uricase, glucose oxidase, glucose dehydrogenase, hexokinase, ascorbic acid oxidase, ascorbic acid dehydrogenase, diaphorase, and the like.

While in the first example and in the reference example BSA is adopted as the solubilized protein, the same effects can be achieved also when egg albumin, gelatin, collagen, or the like is adopted. It can be determined that the optimum value of the additive amount of the solubilized protein is 0.004mg per enzyme 1U or 0.007mg per one sensor.

While in the first example and in the reference example the three-electrode-system biosensor is described, a two-electrode-system biosensor may be used.

### APPLICABILITY IN INDUSTRY

A biosensor of the present invention can be utilized as a highly precise enzyme sensor which can reduce the influence by hematocrit as well as the influence by temperature.

## Claims

1. A biosensor for measuring the concentration of a specific component in a sample solution, wherein electrodes including at least a working electrode and a counter electrode are provided on an insulating substrate,
a solubilized protein is contained in a reagent layer including a reagent which reacts specifically with the specific component in the sample solution,
the solubilized protein is bovine serum albumin, egg albumin, gelatin, or collagen
the reagent layer includes at least an enzyme and an electron carrier, and said reagent layer is formed on the electrodes, or formed so that the electrodes are disposed in a diffusion area in which the reagent of the reagent layer is dissolved in the sample solution to be diffused, and
the enzyme is glucose dehydrogenase having flavin adenine dinucleotide as a coenzyme

2. A biosensor as defined in Claim 1, wherein the amount of the solubilized protein contained is within a range of 0.0004 to 0.008 mg per enzyme 1U.

3. A biosensor as defined in Claim 1, wherein the amount of the solubilized protein contained is within a range of 0.0007 to 0.014 mg per one sensor.

4. A biosensor as defined in Claim 2, wherein the amount of the solubilized protein contained is within a range of 0.0035 to 0.004 mg per enzyme 1U.

5. A biosensor as defined in Claim 3, wherein the amount of the solubilized protein contained is 0.007 mg per one sensor.

## Patentansprüche

1. Biosensor zum Messen der Konzentration einer spezifischen Komponente in einer Probenlösung, wobei Elektroden, die zumindest eine Arbeitselektrode und eine Zählerelektrode beinhalten, auf einem Isoliersubstrat vorgesehen sind,
ein aufgelöstes Protein in einer Reagenzschicht mit einem Reagens, das spezifisch mit der spezifischen Komponente in der Probenlösung reagiert, enthalten ist,
das gelöste Protein Rinderserumalbumin, Eieralbumin, Gallert oder Kollagen ist,
die Reagenzschicht zumindest ein Enzym und einen Elektronenträger enthält, und die Reagenzschicht auf den Elektroden ausgebildet ist, oder derart ausgebildet ist, dass die Elektroden in einem Diffusionsbereich angeordnet sind, in dem das Reagens der Reagenzschicht in der Probenlösung, die diffundiert werden soll, gelöst ist, und
das Enzym Glucosedehydrase mit Flavin-Adenin-Dinukleotid als Koenzym ist.

2. Biosensor nach Anspruch 1, wobei die Menge des enthaltenen aufgelösten Proteins innerhalb eines Bereichs von 0,0004 bis 0,008 mg pro Enzym 1U liegt.

3. Biosensor nach Anspruch 1, wobei die Menge des enthaltenen aufgelösten Proteins innerhalb eines Bereichs von 0,0007 bis 0,014 mg pro einem Sensor liegt.

4. Biosensor nach Anspruch 2, wobei die Menge des enthaltenen aufgelösten Proteins innerhalb eines Bereichs von 0,0035 bis 0,004 mg pro Enzym 1U liegt.

5. Biosensor nach Anspruch 3, wobei die Menge des enthaltenen aufgelösten Proteins 0,007 mg pro einem Sensor beträgt.

## Revendications

1. Biocapteur permettant de mesurer la concentration d'un composant spécifique dans une solution de dosage, où des électrodes comportant au moins une électrode de travail et une contre-électrode sont prévues sur un substrat isolant,
une protéine solubilisée est contenue dans une couche de réactif comportant un réactif qui réagit spécifiquement avec le composant spécifique dans la solution de dosage,
la protéine solubilisée est l'albumine de sérum bovin, blanc d'oeuf, gélatine, ou collagène,
la couche de réactif comporte au moins une enzyme et un porteur d'électrons, et ladite couche de réactif est formée sur les électrodes, ou formée de sorte que les électrodes soient disposées dans une zone de diffusion où le réactif de la couche de réactif est dissous dans la solution de dosage à diffuser, et
l'enzyme est la glucose déshydrogénase ayant en tant que coenzyme la flavine adénine dinucléotide.

2. Biocapteur selon la revendication 1, dans lequel la quantité de la protéine solubilisée contenue se trouve dans une plage allant de 0,0004 à 0,008 mg par 1U d'enzyme.

3. Biocapteur selon la revendication 1, dans lequel la quantité de la protéine solubilisée contenue se trouve dans une plage allant de 0,0007 à 0,014 mg par un capteur.

4. Biocapteur selon la revendication 2, dans lequel la quantité de la protéine solubilisée contenue se trouve dans une plage allant de 0,0035 à 0,004 mg par 1U d'enzyme.

5. Biocapteur selon la revendication 3, dans lequel la quantité de la protéine solubilisée contenue est de 0,007 mg par un capteur.
